# Europäisches Patentamt

## European Patent Office

## Office européen des brevets

(11) Veröffentlichungsnummer: **0 069 113**
**B1**

(12) **EUROPÄISCHE PATENTSCHRIFT**

(45) Veröffentlichungstag der Patentschrift:
**26.08.87**

(51) Int. Cl.⁴: **A 61 B 5/00**, A 61 B 5/02, A 61 B 5/04

(21) Anmeldenummer: **82890088.6**

(22) Anmeldetag: **14.06.82**

(54) **Messsonde zur Überwachung eines Kindes während der Geburt.**

(30) Priorität: **16.06.81 AT 2678/81**

(43) Veröffentlichungstag der Anmeldung:
**05.01.83 Patentblatt 83/1**

(45) Bekanntmachung des Hinweises auf die Patenterteilung:
**26.08.87 Patentblatt 87/35**

(84) Benannte Vertragsstaaten:
**BE CH DE FR GB IT LI NL SE**

(56) Entgegenhaltungen:
EP - A - 0 039 443
DE - A - 2 619 471
DE - A - 2 749 048
DE - A - 2 913 048
DE - A - 2 930 663
DE - B - 1 087 752
FR - A - 2 315 085
US - A - 4 217 910

PROCEEDINGS OF THE 23rd ANNUAL CONFERENCE ON ENGINEERING IN MEDICINE AND BIOLOGY, Washington, 16.-19. November 1970, Band 12, Seite 85, Teil 7.4, New York, USA M.R. NEUMAN: A telematry system for fetal intensive care during labor and delivery"
MEDICAL AND BIOLOGICAL ENGINEERING, Band 14, Nr. 3, Mai 1976, Seiten 361-364, Stevenage, GB. T. YAMAZAKI et al.: "A modified internal temperature measurement device"

(73) Patentinhaber: **Innova Wiener Innovationsgesellschaft m.b.H., Beatrixgasse 1, A-1030 Wien III (AT)**

(72) Erfinder: **Rudelstorfer, Rudolf, Dr., Steckhovengasse 20/3/4, A-1130 Wien XIII (AT)**
Erfinder: **Simbrunner, Georg, Dr., Schwarzspanierstr.15/215, A-1090 Wien IX (AT)**

(74) Vertreter: **Krause, Ernst, Dipl.-Ing. et al, Dipl.- Ing. Krause, Ernst Dipl. Ing. Casati, Wilhelm Patentanwälte Amerlingstrasse 8, A-1061 Wien (AT)**

## Beschreibung

Die Erfindung betrifft eine Messsonde zur Überwachung eines Kindes während der Geburt, welche zumindest eine auf einem vorzugsweise scheibenförmigen Trägerkörper angeordnete, elektrisch leitende Einstichelektrode aufweist, mit welcher der Trägerkörper an der Kopfschwarte oder dem bei der Geburt vorangehenden Kindesteil befestigbar ist und die zur fötalen EKG-Abnahme dient.

Derartige bekannte Messsonden sind im Einsatz nachteilig, weil die Anzahl der von ihnen abgenommenen Daten für eine einwandfreie Diagnose von während des Geburtsvorganges auftretenden Störungen im Organismus des Kindes oder der Mutter oft nicht ausreichend ist. Ferner sind die bekannten Messsonden nicht ohne weiteres ausbaufähig.

Aus der DE-A 2 619 471 ist eine Messsonde zur intauterinen elektrischen Messung der Oberflächentemperatur bekannt, bestehend aus einem scheibenförmigen Körper aus Material geringer Wärmeleitfähigkeit, in dem auf den beiden gegenüberliegenden Scheibenflächen jeweils ein Wärmefühler angeordnet ist. Diese Anordnung erlaubt bei einer Geburt sowohl die Oberflächentemperatur des Kindes als auch die Temperatur der Gebärmutteroberfläche zu bestimmen, indem die Sonde zwischen diese Oberfläche und das Kind einschiebbar ist und die Temperaturdifferenz an einer festen Stelle messbar ist. Eine Messung des Wärmeabflusses vom kindlichen Kopf während der Geburt ist jedoch nicht vorgesehen.

Ziel der Erfindung ist es, eine konstruktiv einfach aufgebaute, betriebssichere, wenig störungsanfällige Messsonde zu erstellen, mit der eine eindeutige Aussage über den Zustand des Kindes während der Geburt erreicht werden kann.

Dieses Ziel wird bei einer Messsonde der eingangs genannten Art dadurch erreicht, dass am Trägerkörper ein Wärmeflussabnehmer, der in bekannter Weise aus einer Wärmeflussplatte und aus zwei an den gegenüberliegenden Seiten der Platte befestigten und gegeneinander geschalteten Thermoelementen besteht, zur Messung des Wärmeabflusses vom kindlichen Kopf bzw. vom vorangehenden Körperteil angeordnet ist, welcher Wärmeflussabnehmer nach der Befestigung des Trägerkörpers am Kind in Kontakt mit der Haut des Kindes steht.

Anhand der Zeichnung werden die Erfindung sowie weitere Einzelheiten und Vorteile derselben beispielsweise näher erläutert. Es zeigen:

Fig. 1 eine Ansicht der in Kontakt mit der Haut stehenden Fläche einer Messsonde,

Fig. 2 eine Ansicht der von der Haut abgewandten Seite der Messsonde,

Fig. 3 einen Mittellängsschnitt durch die Messsonde,

Fig. 4 ein Schaubild eines Führungsrohres für die Messsonde, und

Fig. 5 ein Schaltschema eines Wärmeflussabnehmers.

Bisher wurden bei der Geburtenüberwachung im wesentlichen drei Methoden bzw. Messungen eingesetzt, und zwar erstens das fötale Elektrokardiogramm (EKG), zweitens die extra- bzw. intrauterine Druckmessung, (insbesondere die Stärke der Wehen) und drittens die Analyse des Sauerstoffpartialdruckes und des pH-Wertes des Blutes, entweder durch direkte Blutentnahme aus der Kopfhaut oder durch Messsonden, welche an bzw. in der Kopfhaut fixiert werden. Hauptsächlicher Nachteil dieser Überwachungsmethoden ist eine nicht immer richtige Anzeige einer Gefahr für den Fötus, und folglich wird die Geburt öfter, als es notwendig erscheint, durch Instrumente (Zangen) oder Schnittentbindung (Kaiserschnitt) beendet.

Die erfindungsgemässe Messsonde bietet eine Reihe von Vorteilen gegenüber den konventionellen Überwachungsmethoden. Die erfindungsgemässe Messsonde enthält neben den herkömmlichen Messvorrichtungen für ein fötales EKG und für eine intrauterine Druckmessung einen Wärmeflussabnehmer, mit dem Aussagen über den Stoffwechsel und die Blutzirkulation des Kopfes, vor allem des Gehirns, gemacht werden können. Diese Überwachung unter der Geburt zeigt an, ob das Neugeborene im Geburtskanal tatsächlich beeinträchtigt ist, während dem EKG und den intrauterinen Druckmustern (Cardiotokographie) vor allem eine Warnfunktion zukommt. Solange nämlich eine normale Stoffwechsellage und Zirkulation des Kopfes, insbesondere des Gehirns besteht, wird eine bestimmte Wärmemenge vom Kopf an die Umgebung abgegeben. Wird nun beispielsweise die Blutzirkulation zum Kopf beeinträchtigt, wobei aber meistens für längere Zeit durch Anpassungsmechanismen der Stoffwechsel normal bleibt, so kommt es zu einer vermehrten Wärmeabgabe vom Kopf, da ein Teil der produzierten Wärmemenge aus dem Stoffwechsel nicht mehr durch die Blutzirkulation abgeführt werden kann.

Bei einem Neugeborenen mit hypoplastischem Linksherz, das heisst mit einer stark erniedrigten Zirkulation, kann z.B. der Stoffwechsel durch das Medikament Prostaglandin erhöht werden, und es kommt dann zu einer Erhöhung des Wärmeabflusses vom Kopf des Neugeborenen, weil man bei einem Neugeborenen mit hypoplastischem Linksherz annehmen kann, dass sich die Zirkulation kaum mehr erhöhen lässt.

Bei einem Neugeborenen, das gesund ist und das Medikament Alupent bekommt, erhöht sich ebenfalls der Stoffwechsel. Es kommt wie zuvor zur Erhöhung des Wärmeabflusses bzw. der Wärmeabgabe vom Kopf, wobei man auf Grund der Physiologie annehmen kann, dass die Blutzirkulation zum Kopf sich kaum ändert.

Ein erhöhter Wärmeabfluss vom Kopf kommt also dann vor, wenn entweder weniger Wärme durch die Zirkulation abtransportiert oder mehr Wärme durch den Stoffwechsel produziert wird. Letzteres kommt aber nur unter artifiziellen Bedingungen vor, z.B. bei Medikamentenverabreichung, so dass eine Erhöhung des Wärmeabflusses immer eine Zirkulationsstörung andeutet. Kommt es zu einer Verminderung des Stoffwechsels des Kopfes bzw. Gehirns, so wird weniger Wärme vom Kopf abgegeben, wobei die Wärmemenge auch mit der Herzfrequenz korreliert.

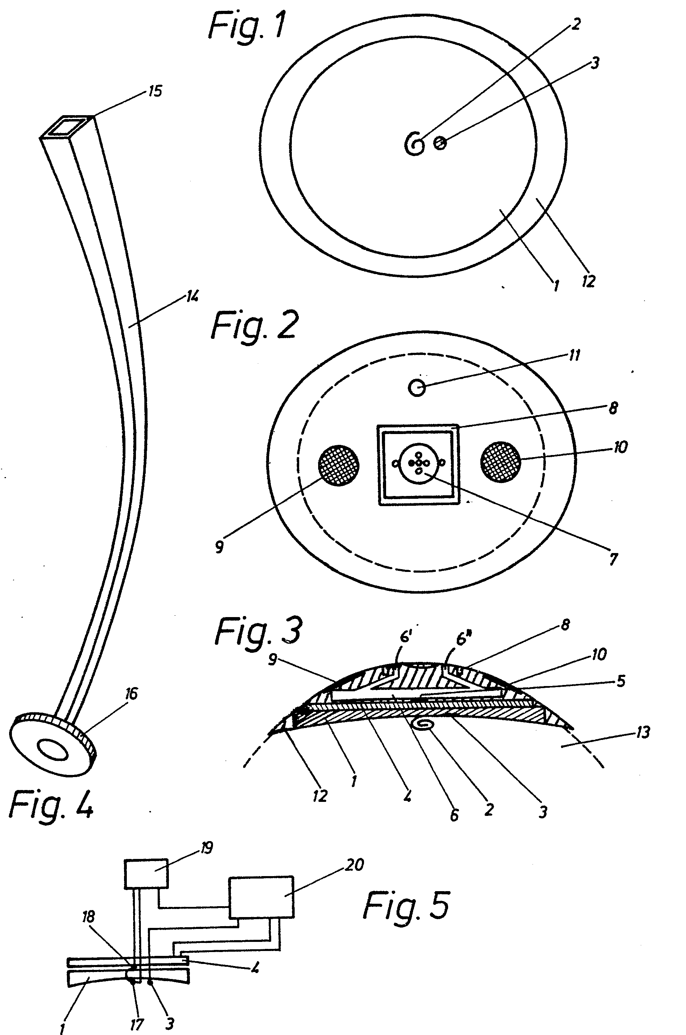

Fig. 1 zeigt die am Körper des Kindes bzw. der Kopfhaut anliegende Seite eines vorzugsweisen scheibenförmigen Trägerkörpers 12 der Messsonde. Der überwiegende Teil dieser Kontaktfläche des Trägerkörpers 12 wird von einem Wärmeflussabnehmer 1 gebildet. In der Mitte ist eine gegen ihn isolierte Einstichelektrode 2 vorgesehen. Beim Ansetzen an der Kopfhaut wird durch eine Drehung die Einstichelektrode 2 in die Kopfschwarte eingestochen und der Trägerkörper 12 verankert. Es können auch mehrere Elektroden 2 vorgesehen sein. Derartige aus elektrisch leitendem Material hergestellte Elektroden 2 sind als Hon'sche Spiralen bekannt und dienen gleichzeitig zur Abnahme der Herzfrequenz des Kindes.

Die erfindungsgemässe Messsonde kann mit den Einstichelektroden 2 am Kopf des Kindes oder auch an einem allenfalls anderen vorangehenden Körperteil befestigt werden, liefert aber in beiden Fällen Werte, welche in nahezu gleicher Weise einen Schluss auf den Zustand des Kindes zulassen. Im folgenden wird unter dem Begriff «Kopf» auch immer ein anderer vorangehender Körperteil des Kindes verstanden.

In der Einstichelektrode 2, oder unmittelbar daneben, um guten Kontakt mit der Haut zu haben, ist ein Thermistor 3 vorgesehen, der zur Messung der Hauttemperatur dient.

Fig. 3 zeigt einen Mittellängsschnitt durch den Trägerkörper 12; dieser ist entsprechend der Krümmung des kindlichen Kopfes 13 auf der Innenseite konkav und auf der anderen Seite konvex geformt und hat einen Radius von zirka 1,5 cm und eine Höhe von 4–6 mm.

Die konkave Seite des Trägerkörpers 12 trägt den Wärmeflussabnehmer 1, in dessen Mitte sich die Hon'sche Einstichelektrode 2 zur Registrierung der kindlichen Herzfrequenz und zur Befestigung des Trägerkörpers 12 befindet. Oberhalb des Wärmeflussabnehmers 1 befindet sich eine Heizfolie bzw. -platte 4, und über ihr ein weiterer Thermistor 5, der in der Mitte des Trägerkörpers 12 gelegen ist.

Der Wärmeflussabnehmer 1 weist zwei Thermoelemente 17, 18 auf (Fig. 5), die gegeneinander geschaltet sind. Die Schweissperle bzw. Verbindungsstelle des einen Thermoelementes 17 steht mit der Kopfhaut in Kontakt, die des anderen Thermoelementes 18 mit der Heizvorrichtung bzw. dem der Haut abgekehrten Bereich des Trägerkörpers.

Die Temperaturdifferenz zwischen den beiden Thermoelementen 17, 18 stellt ein Mass für den Wärmeabfluss aus dem Kopf bzw. aus dem Körperteil dar, an dem der Trägerkörper 12 befestigt ist.

Es ist möglich, als Wärmeflussabnehmer 1 eine konkave Platte vorzusehen, deren Temperatur an beiden Seiten mittels gegeneinander geschalteten Thermoelementen 17, 18 gemessen wird, deren Ausgangssignal der vom Kopf abgegebenen bzw. der durch die Platte hindurchgehenden Wärmemenge proportional ist und einer Vergleichsschaltung 19 zugeführt ist. In der Vergleichsschaltung 19 erfolgt ein Vergleich des gemessenen Wärmeflusswertes mit vorgegebenen Werten, um festzustellen, ob der Wärmefluss bedrohliche Werte annimmt; ein dem Wärmeabfluss entsprechendes Signal wird der Auswerteeinheit 20 zugeführt und dort angezeigt, ebenso wie das Ergebnis des Vergleichs, ob der Wärmeabfluss gefährliche Werte angenommen hat oder nicht.

Ferner weist die Auswerteeinheit 20 eine Regeleinheit für die Heizeinrichtung 4 auf, um entsprechend der «zero-heatflux»-Methode die Körperteilinnentemperatur oder Gehirntemperatur bestimmen zu können. Dazu wird mit der Heizvorrichtung 4 dem Trägerkörper 12 bzw. der aussenliegenden Seite des Wärmeflussabnehmers 1 Wärme zugeführt, bis diese Seite des Wärmeflussabnehmers 1 die gleiche Temperatur wie seine Kontaktfläche aufweist. Dies ist durch das Absinken seines Ausgangssignales bzw. das der Thermoelemente 17, 18 auf Null in der Vergleichsschaltung 19 feststellbar, die über eine entsprechende Leitung mit der Regeleinheit der Auswerteeinheit 20 verbunden ist.

Die Temperatur an der Kontaktfläche erhöht sich hiebei jedoch durch die vom Kopfinneren an die Kopfhaut bzw. vom Körperteilinneren an die Haut geleitete Wärme auf den im Gehirn bzw. Körperteilinneren herrschenden Wert, da keine Wärme mehr abgeleitet wird. Mit dem Thermistor 3 ist in diesem Fall ein Temperaturwert messbar, welcher der tiefen Gehirntemperatur bzw. Körperteilinnentemperatur entspricht und an der Auswerteeinheit 20 anzeigbar und/oder registrierbar ist. Auch plattenförmige Thermoelemente können im Wärmeflussabnehmer eingesetzt werden, wobei die Grösse der Platten der gewünschten Fläche des Wärmeflussabnehmers 1 entsprechen kann.

Zur Verstärkung des Wärmeabflusses vom Kopf oder einem anderen Körperteil ist oberhalb der Heizeinrichtung 4 zumindest ein Kühlhohlraum 6 vorgesehen, in dem ein Kühlmittel zirkulierbar ist, das über Leitungen 6' bzw. 6'' zu- und abgeführt wird. Je tiefer die Temperatur des Kühlmittels bzw. dieses Teiles des Trägerkörpers 12 ist, umso grösser wird die Temperaturdifferenz zur Hautoberfläche und umso mehr nimmt die Wärmeabgabe an der Hautoberfläche zu. Wird die Temperaturdifferenz grösser (um einige °C), so kann der Wärmefluss genauer gemessen und eine genauere Diagnose erstellt werden. Die Art der Kühleinrichtung 6 ist an sich beliebig. Anstelle eines Kühlsystems mit zirkulierendem Kühlmittel kann auch eine elektrische Kühleinrichtung basierend auf Halbleiterkühlelementen vorgesehen sein, deren Wirkung auf dem Peltiereffekt beruht, d.h. durch Stromzufuhr kann die Temperatur der Kühlelemente herabgesetzt werden. Ein Thermistor 5 ist unterhalb der Kühleinrichtung im Trägerkörper 12 angeordnet, um die abgesenkte Temperatur des Trägerkörpers zu messen.

Auf der konvexen Seite des Trägerkörpers 12 befindet sich ein Kabelauslass 7 (Fig. 2) für die notwendigen Leitungs- und Messkabel und die Leitungen der Kühlflüssigkeit. Der Kabelauslass wird von einer quadratisch verlaufenden Rinne 8 umgeben, die zur Aufnahme eines Führungsrohres 14 dient. Dieses Führungsrohr 14 (Fig. 4) ist leicht gebogen und etwa 25 cm lang. Sein Radius beträgt etwa 0,5 cm, es ist aus steifem Kunststoff gefertigt. Ein Ende 15 des Führungsrohres 14 ist quadratisch geformt und passt in die Rinne 8 des Trägerkörpers 12. Das andere Ende des Führungsrohres 14 besitzt eine radförmige Drehvorrichtung 16 zur Drehung des Führungsrohres (14) um seine Längsachse, wodurch der Trägerkörper 12 mit der Einstichelektrode 2 befestigt werden kann. Im Inneren des Führungsrohres 14 verlaufen die Kabel zu der Auswerteeinrichtung 20 bzw. zur Vergleichsschaltung 19.

An der Peripherie der dem Kind abgewandten Seite des Trägerkörpers 12 ist eine scheibenförmige Metallplatte 9 als indifferente Elektrode für die Registrierung der kindlichen Herzfrequenz eingebaut (Fig. 2). Ihr gegenüber befindet sich ein weiterer Thermistor 10 zur Messung der Umgebungstemperatur und eine Öffnung 11 für eine Einrichtung zur intrautinären Druckmessung. Diese Drucksignale können als Korrekturwerte für die Wärmeabflusssignale verwendet werden.

Die Messsonde ist wasserdicht, entsprechend elektrisch isoliert und sterilisierbar.

**Patentansprüche**

1. Messsonde zur Überwachung eines Kindes während der Geburt, welche zumindest eine auf einem vorzugsweise scheibenförmigen Trägerkörper (12) angeordnete, elektrisch leitende Einstichelektrode (2) aufweist, mit welcher der Trägerkörper an der Kopfschwarte oder dem bei der Geburt vorangehenden Kindesteil befestigbar ist und die zur fötalen EKG-Abnahme dient, dadurch gekennzeichnet, dass am Trägerkörper (12) ein Wärmeflussabnehmer (1; 17, 18), der in bekannter Weise aus einer Wärmeflussplatte (1) und aus zwei an den gegenüberliegenden Seiten der Platte (1) befestigten und gegeneinander geschalteten Thermoelementen (17, 18) besteht, zur Messung des Wärmeabflusses vom kindlichen Kopf (13) bzw. vom vorangehenden Körperteil angeordnet ist, welcher Wärmeflussabnehmer (1; 17, 18) nach der Befestigung des Trägerkörpers (12) am Kind in Kontakt mit der Haut des Kindes steht.

2. Messsonde nach Anspruch 1, dadurch gekennzeichnet, dass in der Einstichelektrode (2) oder in ihrer unmittelbaren Nähe ein Thermistor (3) zur Messung der Temperatur der Haut des Kindes vorgesehen ist, dessen Ausgangssignal an einer Auswerteeinheit (20) anzeigbar und/oder registrierbar ist.

3. Messsonde nach Anspruch 1 oder 2, dadurch gekennzeichnet, dass an der dem Kind abgewandten Seite des Wärmeflussabnehmers (1; 17, 18) eine Heizvorrichtung (4), z.B. eine Heizfolie oder -platte angeordnet bzw. an diesen angelegt ist.

4. Messsonde nach Anspruch 3, dadurch gekennzeichnet, dass die Heizeinrichtung (4) an eine Regeleinheit angeschlossen ist, mit der die Wärmeabgabe der Heizeinrichtung (4) zum Trägerkörper (12) bzw. zum Wärmeflussabnehmer (1; 17, 18) in Abhängigkeit vom Wert des Ausgangssignales des Wärmeflussabnehmers (1) regelbar ist.

5. Messsonde nach Anspruch 4, dadurch gekennzeichnet, dass die vorzugsweise in der Auswerteeinheit (20) angeordnete Regeleinheit mit einer Vergleichsschaltung (19) verbunden ist, der das Ausgangssignal des Wärmeflussabnehmers (1; 17, 18) zugeführt ist und in der dieses Signal mit einem vorgegebenen Signalwert, vorzugsweise dem Wert Null, vergleichbar ist, und dass bei Erreichen des vorgegebenen Signalwertes das vom Thermistor (3) gemessene Temperatursignal als Gehirn- bzw. Körperteilinnentemperatur an der Auswerteeinheit (20) anzeigbar und/oder registrierbar ist.

6. Messsonde nach einem der Ansprüche 1 bis 5, dadurch gekennzeichnet, dass zur Verstärkung des Wärmeflusses im Trägerkörper (12) an der dem Kind abgewandten Seite des Heizeinrichtung (4) eine Kühleinrichtung (6), z.B. ein Hohlraum oder Kanäle, in denen Kühlflüssigkeit zirkulierbar ist oder ein Halbleiterkühlelement bzw. Peltierelement vorgesehen ist.

7. Messsonde nach Anspruch 6, dadurch gekennzeichnet, dass unterhalb der Kühleinrichtung (6), vorzugsweise in der Mitte des Trägerkörpers (12) ein Thermistor (5) angeordnet ist.

8. Messsonde nach einem der Ansprüche 1 bis 7, dadurch gekennzeichnet, dass an der dem Kind abgewandten Seite des Trägerkörpers (12) eine indifferente Elektrode (9) zur Registrierung der kindlichen Herzfrequenz angeordnet ist.

9. Messsonde nach einem der Ansprüche 1 bis 8, dadurch gekennzeichnet, dass an der dem Kind abgewandten Seite des Trägerkörpers (12) ein weiterer Thermistor (10) zur Messung der Umgebungstemperatur angeordnet ist.

10. Messsonde nach einem der Ansprüche 1 bis 9, dadurch gekennzeichnet, dass an der dem Kind abgewandten Seite des Trägerkörpers (12) eine quadratisch verlaufende, vorzugsweise einen Kabelauslass umschliessende Rinne (8) vorgesehen ist, in welche das Ende (15) eines Führungsrohres (14) einsteckbar ist, durch welches die Messkabel und gegebenenfalls die Leitungen für die Kühlflüssigkeit durchgeführt sind.

11. Messsonde nach einem der Ansprüche 1 bis 10, dadurch gekennzeichnet, dass die dem Kopf (13) zugewandte Seite des Trägerkörpers (12) konkav und seine andere Seite konvex geformt ist, wobei der Durchmesser des Trägerkörpers (12) etwa 30 mm und seine Höhe etwa 4–6 mm beträgt.

12. Messsonde nach einem der Ansprüche 1 bis 11, dadurch gekennzeichnet, dass nahezu die gesamte, am Kopf (13) anliegende Fläche des Trägerkörpers (12) von dem konkav gekrümmten Wärmeflussabnehmer (1; 17, 18) gebildet ist.

13. Messsonde nach einem der Ansprüche 1 bis 12, dadurch gekennzeichnet, dass auf der dem Kopf (13) abgewandten Seite des Trägerkörpers (12) eine Druckmesseinrichtung vorgesehen ist, die in einer Öffnung (11) des Trägerkörpers (12) angeordnet ist.

14. Messsonde nach einem der Ansprüche 1 bis 13, dadurch gekennzeichnet, dass die Signale der einzelnen, am Trägerkörper (12) angeordneten Messeinrichtungen an die Auswerteeinheit (20) geführt und dort anzeigbar und/oder registrierbar sind.

## Claims

1. A sensing probe for monitoring an infant during birth, comprising at least one electrically conducting puncturing electrode (2), which is provided on a preferably disc-shaped carrier body (12) and with which the carrier body os adapted to be secured to the scalp or that portion of the infant which emerges first during birth, which electrode is used to take the ECG of the foetus, characterized in that a heat flux pick-up (1; 17, 18) is provided on the carrier body (12) and consists in known manner of a heat flux plate (1) and two thermocouples (17, 18), which are secured to the opposite sides of the plate (1) and are connected in series opposition and serve to measure the heat flux from the head (13) of the infant or from the first-emerging part of the body, said heat flux pick-up (1; 17, 18) being in contact with the skin of the infant when the carrier body (12) has been secured to the infant.

2. A sensing probe according to claim 1, characterized in that a thermistor (3) for measuring the temperature of the skin of the infant is provided in or close to the puncturing electrode (2), the output signal of which thermistor being adapted to be indicated and/or recorded by an interpreting unit (20).

3. A sensing probe according to claim 1 or 2, characterized in that a heater (4), e.g., a heating sheet or plate, is arranged on and/or in contact with the heat flux pick-up (1; 17, 18) on that side thereof which is remote from the infant.

4. A sensing probe according to claim 3, characterized in that the heater (4) is connected to an automatic controller for automatically controlling the transfer of heat from the heater (4) to the carrier body (12) and/or to the heat flux pick-up (1; 17, 18) in dependence on the value of the output signal of the heat flux pick-up (1).

5. A sensing probe according to claim 4, characterized in that the automatic controller, which is preferably incorporated in the interpreting unit (20), is connected to a comparator circuit (19), to which the output signal of the heat flux pick-up (1; 17, 18) is delivered and which is adapted to compare that signal with a predetermined signal value, preferably the value zero, and the interpreting unit (20) is adapted to indicate and/or record the temperature signal measured by the thermistor (3) as a brain or body part internal temperature when the predetermined signal value has reached the predetermined value.

6. A sensing probe according to any of claims 1 to 5, characterized in that the flux in the carrier body (12) is increased by a cooler (6), e.g., a cavity or passages permitting of the circulation of a liquid coolant, or by a semiconductor cooling element or a Peltier element, provided on that side of the heater (4) which is remote from the infant.

7. A sensing probe according to claim 6, characterized in that a thermistor (5) is arranged below the cooler (6), preferably at the centre of the carrier body (12).

8. A sensing probe according to any of claims 1 to 7, characterized in that an indifferent electrode (9) for detecting the heart rate of the infant if provided on that side of the carrier body (12) which is remote from the infant.

9. A sensing probe according to any of claims 1 to 8, characterized in that another thermistor (10) for measuring the ambient temperature is provided on that side of the carrier body (12) which is remote from the infant.

10. A sensing probe according to any of claims 1 to 9, characterized in that a groove (8) which has a square configuration and preferably encloses a cable outlet is provided on that side of the carrier body (12) which is remote from the infant, the end (15) of a guide tube (14) is adapted to be fitted into said groove, and the sensing cables and optionally the lines for the liquid coolant extend through said guide tube.

11. A sensing probe according to any of claims 1 to 10, characterized in that the carrier body (12) is concave on that side which faces the head (13) and is convex on the other side and has a diameter of about 30 mm and a height of about 4 to 6 mm.

12. A sensing probe according to any of claims 1 to 11, characterized in that almost the entire surface with which the carrier body (12) contacts the head (13) is constituted by the concavely curved heat flux pick-up (1; 17, 18).

13. A sensing probe according to any of claims 1 to 12, characterized in that a pressure sensor is arranged in an opening (11) of the carrier body (12) on that side thereof which is remote from the head (13).

14. A sensing probe according to any of claims 1 to 13, characterized in that the signals from the several sensing means provided on the carrier body (12) are delivered to the interpreting unit (20) and the latter is adapted to indicate and/or record said signals.

## Revendications

1. Capteur de mesure pour la surveillance d'un nouveau-né pendant l'accouchement, qui présente au moins une électrode-aiguille (2) électroconductrice disposée sur un corps support (12) de préférence en forme de disque, par laquelle le corps support peut être fixé sur le cuir chevelu ou la partie du corps du nouveau-né sortant la première lors de la naissance, et qui sert à l'électrocardiographie foetale, caractérisé en ce qu'est disposé sur le corps support (12) un récepteur de flux de chaleur (1; 17, 18), qui est constitué d'une manière connue d'une plaque de flux de chaleur (1) et de deux thermoéléments (17, 18) fixés sur les côtés opposés de la plaque (1) et montés en opposition, et qui sert à mesurer le dégagement de chaleur de la tête (13) du nouveau-né ou selon le cas de la partie du corps du nouveau-né sortant la première lors de la naissance, ledit récepteur de flux de chaleur (1; 17, 18) étant en contact avec la peau du nouveau-né une fois le corps support (12) fixé sur le nouveau-né.

2. Capteur de mesure selon la revendication 1, caractérisé en ce qu'une thermistance (3) est prévue dans l'électrode-aiguille (2) ou dans son voisinage immédiat pour mesurer la température de la peau du nouveau-né, thermistance dont le signal de sortie peut être affiché et/ou enregistré à une unité d'évaluation (29).

3. Capteur de mesure selon la revendication 1 ou 2, caractérisé en ce qu'un élément chauffant (4), tel par exemple qu'une feuille ou qu'une plaque chauffante, est disposé sur le côté du récepteur de flux de chaleur (1; 17, 18) éloigné du nouveau-né, ou est appliqué contre ledit récepteur de flux de chaleur.

4. Capteur de mesure selon la revendication 3, caractérisé en ce que l'élément chauffant (4) est relié à une unité de régulation, qui permet de régler le dégagement de chaleur de l'élément chauffant (4) vers le corps support (12) ou encore vers l'évacuateur de flux de chaleur (1; 17, 18), en fonction de la valeur du signal de sortie du récepteur de flux de chaleur (1).

5. Capteur de mesure selon la revendication 4, caractérisé en ce que l'unité de régulation, disposée de préférence dans l'unité d'évaluation (20), est reliée à un montage comparateur (19) auquel est amené le signal de sortie du récepteur de flux de chaleur (1; 17, 18), et dans lequel ce signal peut être comparé à une valeur de signal prédéterminée, de préférence la valeur zéro, et en ce que lors de l'atteinte de la valeur de signal prédéterminée, le signal de température mesuré par la thermistance (3) peut être affiché et/ou enregistré à l'unité d'évaluation (20) comme température interne de l'encéphale ou selon le cas de ladite partie du corps du nouveau-né.

6. Capteur de mesure selon une quelconque des revendications 1 à 5, caractérisé en ce que, pour renforcer le flux de chaleur dans le corps support (12), il est prévu sur le côté de l'élément chauffant (4) éloigné du nouveau-né un élément réfrigérant (6), tel par exemple qu'une cavité ou que des canaux dans lesquels peut circuler un liquide réfrigérant, ou qu'un élément réfrigérant à semiconducteur ou encore qu'un élément Peltier.

7. Capteur de mesure selon la revendication 6, caractérisé en ce qu'une thermistance (5) est disposée en dessous de l'élément réfrigérant (6), et de préférence au milieu du corps support (12).

8. Capteur du mesure selon une quelconque des revendications 1 à 7, caractérisé en ce qu'une électrode indifférente (9) est disposée sur le côté du corps support (12) éloigné du nouveau-né, pour mesurer la fréquence cardiaque du nouveau-né.

9. Capteur de mesure selon une quelconque des revendications 1 à 8, caractérisé en ce qu'une nouvelle thermistance (10) est disposée sur le côté du corps support (12) éloigné du nouveau-né, pour mesurer la température ambiante.

10. Capteur de mesure selon une quelconque des revendications 1 à 9, caractérisé en ce qu'il est prévu sur le côté du corps support (12) éloigné du nouveau-né une rigole (8) s'étendant en carré et de préférence entourant une sortie de câbles, rigole dans laquelle peut être emboîtée l'extrémité d'un tube de guidage (14) par lequel sont amenés les câbles de mesure et les éventuelles conduites de liquide réfrigérant.

11. Capteur de mesure selon une quelconque des revendications 1 à 10, caractérisé en ce que le côté du corps support (12) tourné vers la tête (13) est de forme concave et l'autre côté convexe, le diamètre du corps support (12) étant approximativement égal à 30 mm, et sa hauteur à 4–6 mm.

12. Capteur de mesure selon une quelconque des revendications 1 à 11, caractérisé en ce que la quasi-totalité de la surface du corps support (12) qui est appliquée contre la tête (13) est constituée par le récepteur de flux de chaleur (1; 17, 18), qui est courbé de manière concave.

13. Capteur de mesure selon une quelconque des revendications 1 à 12, caractérisé en ce qu'il est prévu sur le côté du corps support (12) éloigné de la tête (13) un élément de mesure de pression, qui est disposé dans une ouverture (11) du corps support (12).

14. Capteur de mesure selon une quelconque des revendications 1 à 13, caractérisé en ce que les signaux des différents éléments de mesure disposés sur le corps support (12) sont amenés à l'unité d'évaluation (20), et peuvent y être affichés et/ou enregistrés.

*Fig. 1*

*Fig. 2*

*Fig. 3*

*Fig. 4*

*Fig. 5*